# EUROPEAN PATENT APPLICATION

(11) **EP 4 847 535 A1**
(43) Date of publication of application: **02.12.2026**
(21) Application number: 25744421.6
(22) Date of filing: 03.01.2025
(51) Int. Cl.: C07K 19/00, C07K 14/745, C12N 15/62, C12N 15/12, C12N 15/63, C12N 15/64, A61K 48/00, A61K 38/37, A61P 7/04, A61P 7/00

(54) **DUAL-SIGNAL PEPTIDE FOR IMPROVING SECRETION LEVEL OF BLOOD COAGULATION FACTOR VIII AND USE THEREOF**

(30) Priority: 23.01.2024 CN 202410097964
(71) Applicant: Shanghai Pinpoint Medical Technology Co., Ltd, Shanghai 201208 (CN)
(72) Inventor: LIANG, Desheng, Shanghai 201208 (CN); TANG, Qiyu, Shanghai 201208 (CN); ZHAO, Junya, Shanghai 201208 (CN); ZHOU, Daixing, Shanghai 201208 (CN)
(74) Representative: Gille Hrabal Partnerschaftsgesellschaft mbB Patentanwälte
(86) International application number: PCT/CN2025/070490
(87) International publication number: WO 2025/156975

(57) **Abstract**

The present disclosure provides a dual-signal peptide. The dual-signal peptide comprises a first signal peptide and a second signal peptide, wherein the first signal peptide comprises an exogenous signal peptide, the second signal peptide comprises an endogenous signal peptide of coagulation factor VIII, with the first signal peptide and the second signal peptide linked in tandem. The dual-signal peptide of the present invention substantially improves both the secretion efficiency and coagulation activity of coagulation factor VIII, significantly increasing the production yield of FVIII, enhancing the industrial production efficiency, further promoting its application in medicaments for treating hemophilia, and contributing to the reduction of treatment costs.

## Description

### CROSS-REFERENCE TO RELATED APPLICATION

The present disclosure claims priority to the prior application with the patent application No. 202410097964.6, entitled "DUAL-SIGNAL PEPTIDE FOR IMPROVING THE SECRETION LEVEL OF COAGULATION FACTOR VIII AND USE THEREOF", and filed with the China National Intellectual Property Administration on January 23, 2024, which is incorporated herein by reference in its entirety.

### TECHNICAL FIELD

The present disclosure relates to the technical field of biosynthesis of coagulation factors, and in particular, to a dual signal peptide for improving the secretion level of coagulation factor VIII and use thereof.

### BACKGROUND

Hemophilia A (HA) is an X-linked recessive genetic disease caused by abnormalities in the gene encoding coagulation factor VIII, resulting in a deficiency or functional defect of coagulation factor VIII . The structure of coagulation factor VIII is composed of several distinct domains, including 3 A domains, 1 B domain, and 2 C domains. Among them, the B domain is highly glycosylated, and deletion of the entire B domain or most of the B domain does not affect the activity of coagulation factor VIII. Studies have shown that, whether for wild-type coagulation factor VIII or recombinantly engineered coagulation factor VIII, the molecular thereof is generally large in size. After introducing an exogenous gene encoding coagulation factor VIII into cells, the expression level of coagulation factor VIII in the cells is relatively low and far below the actual demand. Therefore, promoting the expression and secretion of coagulation factor VIII in cells is one of the technical problems to be solved by those skilled in the art.

A signal peptide, also known as a leader peptide, a signal sequence, a transit peptide, or the like, and abbreviated as SP, is located at the N-terminus (i.e., the amino terminus) of a nascent protein. It generally consists of 5-30 amino acids and is a short peptide chain that directs the transport and secretion of newly synthesized proteins. A signal peptide typically comprises three parts: 1. a positively charged N-terminal region; 2. a central hydrophobic sequence, which is generally mainly composed of neutral amino acids and capable of forming a helical structure, and serves as the main functional region of the signal peptide; and 3. a negatively charged C-terminal region comprising small amino acids, which serves as a cleavage site of the signal peptide and the processing region of the signal peptide. The signal peptide is a signal recognition element associated with the endoplasmic reticulum membrane. Under its guidance, the nascent proteins are translocated into the lumen of the endoplasmic reticulum, after which the signal peptide can be cleaved by the signal peptidase. For secretory proteins, the signal peptide plays an important role in the transport and secretion of the secretory proteins by facilitating intracellular transport, thereby enabling secretion of the secretory proteins outside the cell. For secretory proteins expressed from exogenous genes, in the absence of a signal peptide, the secretion efficiency is poor or absent. When a signal peptide is fused thereto, good secretion can be achieved. However, both secretory proteins and signal peptides are diverse. For the same secretory protein, different signal peptides may result in different secretion efficiencies; and for the same signal peptide, different secretion efficiencies are generally observed when it is used with different secretory proteins.

US5880327A discloses transgenic mammals expressing human coagulation factor VIII. CN109929029A discloses a method for improving high-efficiency expression of recombinant human coagulation factor VIII. However, the secretion level and secretion efficiency of human coagulation factor VIII in these patents are not satisfactory.

### SUMMARY

In view of how to promote the expression and secretion of coagulation factor VIII in cells, the inventors of the present application unexpectedly discovered through experiments that, when coagulation factor VIII is equipped with a dual signal peptide, the secretion level of the coagulation factor VIII in cells can be significantly improved.

In a first aspect, the present disclosure provides a dual signal peptide, comprising a first signal peptide and a second signal peptide, wherein the first signal peptide comprises a heterologous signal peptide, the second signal peptide comprises an endogenous signal peptide of coagulation factor VIII, and the first signal peptide and the second signal peptide are linked in tandem.

In one embodiment of the present disclosure, the dual signal peptide is used for promoting the secretion of coagulation factor VIII and/or improving the activity of coagulation factor VIII.

In a preferred embodiment of the present disclosure, the heterologous signal peptide is one or both of Gaussia luciferase and IgG V.

In one embodiment of the present disclosure, the heterologous signal peptide comprises an amino acid sequence as follows: the amino acid sequence of the heterologous signal peptide is set forth in any one of SEQ ID NO: 4 and SEQ ID NO: 6, or an amino acid sequence having at least 95%, at least 96%, at least 97%, at least 98%, or at least 99% sequence identity to the sequence set forth in any one of SEQ ID NO: 4 and SEQ ID NO: 6 and having substantially the same activity.

In one embodiment of the present disclosure, the endogenous signal peptide has an amino acid sequence set forth in SEQ ID NO: 8, or an amino acid sequence having at least 95%, at least 96%, at least 97%, at least 98%, or at least 99% sequence identity to the sequence set forth in SEQ ID NO: 8 and having substantially the same activity.

In one embodiment of the present disclosure, the first signal peptide and the second signal peptide being linked in tandem comprises a C-terminus of the first signal peptide being linked to an N-terminus of the second signal peptide.

In one embodiment of the present disclosure, a spacer sequence is present or absent between the first signal peptide and the second signal peptide. In one embodiment of the present disclosure, no spacer sequence is present between the first signal peptide and the second signal peptide. That is, the first signal peptide and the second signal peptide are covalently linked by a single peptide bond. In another embodiment, a spacer sequence of one or more amino acids is present between the first signal peptide and the second signal peptide. In principle, there is no limitation on the length of the spacer sequence. In one embodiment of the present disclosure, the spacer sequence between the first signal peptide and the second signal peptide comprises 2-14 amino acids.

In one embodiment of the present disclosure, the dual signal peptide has an amino acid sequence set forth in SEQ ID NO: 11, or an amino acid sequence having at least 95%, at least 96%, at least 97%, at least 98%, or at least 99% sequence identity to SEQ ID NO: 11.

In a second aspect, the present disclosure provides a polypeptide, comprising the dual signal peptide described above and coagulation factor VIII.

In one embodiment of the present disclosure, the coagulation factor VIII comprises modified or unmodified coagulation factor VIII.

In one embodiment of the present disclosure, the coagulation factor VIII is selected from full-length coagulation factor VIII or a variant thereof, or B-domain-deleted coagulation factor VIII or a variant thereof, including but not limited to a BDDF8-CO variant, a BDDF8-SQ variant, a BDDF8-N222 variant, a BDDF8-N6 variant, and the like.

In one embodiment of the present disclosure, the coagulation factor VIII (without the endogenous signal peptide) has an amino acid sequence set forth in SEQ ID NO: 10, or an amino acid sequence having at least 95%, at least 96%, at least 97%, at least 98%, or at least 99% sequence identity to SEQ ID NO: 10 and having substantially the same activity.

In one embodiment of the present disclosure, the C-terminus of the dual signal peptide is linked to an N-terminus of the coagulation factor VIII.

In one embodiment of the present disclosure, a spacer sequence is present or absent between the dual signal peptide and the coagulation factor VIII. In one embodiment of the present disclosure, no spacer sequence is present between the dual signal peptide and the coagulation factor VIII. That is, the dual signal peptide and the coagulation factor are covalently linked by a single peptide bond. In another embodiment, a spacer sequence of one or more amino acids is present between the dual signal peptide and the coagulation factor VIII. In principle, there is no limitation on the length of the spacer sequence. In one embodiment of the present disclosure, the spacer sequence between the dual signal peptide and the coagulation factor VIII comprises 2-14 amino acids.

In an embodiment of the present disclosure, the polypeptide has an amino acid sequence set forth in SEQ ID NO: 12, or an amino acid sequence having at least 95%, at least 96%, at least 97%, at least 98%, or at least 99% sequence identity to SEQ ID NO: 12.

In a third aspect, the present disclosure provides a polynucleotide encoding the dual signal peptide or the polypeptide described above.

In one embodiment of the present disclosure, the polynucleotide sequence encoding the first signal peptide is set forth in any one of SEQ ID NO: 1 and SEQ ID NO: 3, or a nucleotide sequence having at least 95%, at least 96%, at least 97%, at least 98%, or at least 99% sequence identity to the polynucleotide sequence encoding the first signal peptide set forth in any one of SEQ ID NO: 1 and SEQ ID NO: 3.

In one embodiment of the present disclosure, the polynucleotide sequence encoding the second signal peptide is set forth in SEQ ID NO: 7, or a nucleotide sequence having at least 95%, at least 96%, at least 97%, at least 98%, or at least 99% sequence identity to SEQ ID NO: 7.

In one embodiment of the present disclosure, the sequence encoding the coagulation factor VIII (without the endogenous signal peptide) is set forth in SEQ ID NO: 9, or a nucleotide sequence having at least 95%, at least 96%, at least 97%, at least 98%, or at least 99% sequence identity to SEQ ID NO: 9.

In one embodiment of the present disclosure, the polynucleotide sequence encoding the first signal peptide, the polynucleotide sequence encoding the second signal peptide, and the sequence encoding the coagulation factor VIII are operably linked.

In one embodiment of the present disclosure, the polynucleotide comprises, from the 5' end to the 3' end, the polynucleotide sequence encoding the first signal peptide-the polynucleotide sequence encoding the second signal peptide-the sequence encoding the coagulation factor VIII.

In one embodiment of the present disclosure, the polynucleotide has a sequence set forth in SEQ ID NO: 13, or a nucleotide sequence having at least 95%, at least 96%, at least 97%, at least 98%, or at least 99% sequence identity to SEQ ID NO: 13.

In one embodiment of the present disclosure, the polynucleotide further comprises a promoter and a 3' untranslated polynucleotide sequence. In a preferred embodiment of the present disclosure, the 3' untranslated polynucleotide sequence comprises a PolyA tail.

In one embodiment of the present disclosure, the promoter comprises a homologous promoter or a heterologous promoter.

In one embodiment of the present disclosure, the promoter has tissue-preferential expression.

In one embodiment of the present disclosure, the promoter, the polynucleotide, and the 3' untranslated polynucleotide sequence are operably linked.

In one embodiment of the present disclosure, the polynucleotide comprises, from the 5' end to the 3' end, the promoter-the polynucleotide sequence encoding the first signal peptide-the polynucleotide sequence encoding the second signal peptide-the 3' untranslated polynucleotide sequence.

In one embodiment of the present disclosure, the polynucleotide comprises, from the 5' end to the 3' end, the promoter-the polynucleotide sequence encoding the first signal peptide-the polynucleotide sequence encoding the second signal peptide-the sequence encoding the coagulation factor VIII-the 3' untranslated polynucleotide sequence.

In one embodiment of the present disclosure, the polynucleotide comprises, from the 5' end to the 3' end, a CMV promoter-the polynucleotide sequence encoding the first signal peptide-the polynucleotide sequence encoding the second signal peptide-the sequence encoding the coagulation factor VIII-the PolyA tail.

In a fourth aspect, the present disclosure provides an expression vector comprising the polynucleotide described above.

In one embodiment of the present disclosure, the expression vector may be a plasmid or a viral vector. In a preferred embodiment, the plasmid comprises an AAV expression plasmid. The viral vector is selected from the group consisting of AAV vectors, lentiviral vectors, retroviral vectors, adenoviral vectors, and synthetic viral vectors.

In a fifth aspect, the present disclosure provides a host cell comprising one or more of the dual signal peptide, the polypeptide, the polynucleotide, or the expression vector described above.

In one embodiment of the present disclosure, the host cell comprises bacteria, fungi, viruses, animal cells, and/or plant cells.

In a sixth aspect, the present disclosure provides a pharmaceutical composition comprising one or more of the dual signal peptide, the polypeptide, the polynucleotide, the expression vector, and the host cell described above.

In one embodiment of the present disclosure, the pharmaceutical composition further comprises a pharmaceutically acceptable carrier or excipient.

In a seventh aspect, the present disclosure provides use of the dual signal peptide, the polypeptide, the polynucleotide, the expression vector, the host cell, or the pharmaceutical composition described above for manufacturing coagulation factor VIII or manufacturing a medicament for treating hemophilia.

In an eighth aspect, the present disclosure provides a method for secreting coagulation factor VIII, comprising secreting coagulation factor VIII using the dual signal peptide, the polypeptide, the polynucleotide, the expression vector, the host cell, or the pharmaceutical composition described above.

In one embodiment of the present disclosure, the method comprises the following steps: constructing an expression vector containing the polynucleotide encoding the dual signal peptide described above and the sequence encoding coagulation factor VIII, introducing the expression vector into a host cell, and culturing the host cell under conditions allowing expression and secretion of the coagulation factor VIII to obtain the coagulation factor VIII.

In one embodiment of the present disclosure, the method comprises culturing the host cell described above under conditions allowing expression and secretion of the coagulation factor VIII, and isolating the coagulation factor VIII from a cell culture.

Compared with the prior art, the present disclosure has the following beneficial effects:
(1) In the present disclosure, it has been unexpectedly discovered through experiments that a single signal peptide cannot achieve good secretion and expression of coagulation factor VIII, and either no secretion can be detected or the secretion level is relatively low. However, when a dual signal peptide is used for coagulation factor VIII, improved secretion efficiency and coagulation activity are observed. Preferably, when a heterologous signal peptide and an endogenous signal peptide are combined to guide expression and secretion of FVIII, the effect is most significant. More preferably, when a dual signal peptide comprising a heterologous Gluc signal peptide or an IgG V signal peptide and an endogenous signal peptide is used, the expression level of FVIII is significantly increased compared with that of a single signal peptide group (comprising only an endogenous signal peptide or only a heterologous signal peptide), and is up to more than 200 ng/mL.
(2) In the present disclosure, for the problem of secretion level of FVIII in cells, a dual signal peptide structure is selected through experimental optimization, such that the optimal expression and secretion efficiency is achieved. Compared with a single signal peptide, both the secretion efficiency and coagulation activity are substantially improved. In particular, when a dual signal peptide comprising a heterologous Gluc signal peptide or an IgG V signal peptide and an endogenous signal peptide is used, it is also significantly superior to other types of signal peptides, and the coagulation activity is greatly improved. The present disclosure can significantly increase the production yield of FVIII, improve its industrial production efficiency, further promote its application in medicaments for treating hemophilia, and contribute to reducing treatment costs.

### BRIEF DESCRIPTION OF THE DRAWINGS

FIG. 1 is a schematic diagram of exogenous gene expression cassettes carrying a single signal peptide or a dual signal peptide according to the present disclosure.
FIG. 2 shows a comparison of effects of different signal peptides or signal peptide combinations on activity of FVIII expressed and secreted by HEK-293T cells according to Example 2 of the present disclosure.
FIG. 3 shows a comparison of effects of different signal peptides or signal peptide combinations on expression and secretion levels of FVIII in HEK-293T cells according to Example 2 of the present disclosure.

### DETAILED DESCRIPTION

The technical solutions of the present disclosure are further described in detail below with reference to specific examples. It should be understood that the following examples are merely exemplary illustrations and explanations of the present disclosure and should not be construed as limiting the claimed scope of the present disclosure. All techniques implemented on the basis of the content described above of the present disclosure fall within the claimed scope of the present disclosure.

Unless otherwise stated, the raw materials and reagents used in the following examples are all commercially available or can be prepared by known methods. In a specific embodiment of the present disclosure, the secretion level or expression level of coagulation factor VIII is determined using coagulation factor VIII secreted by cells as the detection target, and specifically, the detection results obtained from the supernatant shall prevail.

### Technical Terms

The term "coagulation factor VIII" is also known as FVIII or F8. B-domain-deleted F8: The complete F8 gene encodes full-length factor VIII (FVIII), which comprises six domains, namely A1, A2, B, A3, C1, and C2. The B domain is not essential for the coagulation catalytic activity of FVIII. The B-domain-deleted F8 gene is relatively small, easy to integrate, and encodes FVIII having coagulation activity.

The term "spacer sequence" refers to a nucleotide sequence spacer of 1-100 bp or longer, or an amino acid sequence spacer of 1-100 amino acids or longer. The spacer sequence may be a multiple cloning site, a recombination site, and may comprise a transcription factor binding site, other functional fragments, or the like. The spacer sequence does not affect the function of the dual signal peptide and does not affect the secretion and expression of FVIII. In the present disclosure, the dual signal peptides may be directly linked to each other, and the signal peptide and the secretory protein (such as FVIII) may also be directly linked, or a spacer sequence may be added. Preferably, direct linkage is used both between the dual signal peptides and between the signal peptide and the secretory protein.

In the present disclosure, the promoter and the dual signal peptide are linked via a PacI endonuclease recognition sequence (TTAATTAA), and BDDF8-CO and polyA (pA) are linked via a NotI endonuclease recognition sequence (GCGGCCGC).

The term "signal peptide" refers to a short peptide chain of 5-30 amino acids in a newly synthesized polypeptide chain that directs the protein to the secretory pathway. It is located at the N-terminus of a secretory protein and comprises three parts: a positively charged basic amino-terminal region at the N-terminus; a central hydrophobic sequence composed of neutral amino acids and serving as the main functional region; and a negatively charged C-terminal processing region, which serves as the cleavage site of the signal peptide. Newly synthesized proteins are guided into the lumen of the endoplasmic reticulum by the signal peptide, and the signal peptide sequence is cleaved by the action of a signal peptidase, after which the proteins continue translation while undergoing folding and modification. The signal peptide may be used with a heterologous promoter or with its native promoter. In the present disclosure, the promoter molecule may be operably linked to an endogenous signal peptide or may be operably linked to a heterologous signal peptide.

The term "endogenous signal peptide" refers to a signal peptide derived from the target gene (protein) itself within an organism. In the present disclosure, the endogenous signal peptide comprises the endogenous signal peptide of coagulation factor VIII, which comprises 19 amino acids, the amino acid sequence of which is set forth in SEQ ID NO: 8, or a fragment or a variant thereof.

The term "heterologous signal peptide" refers to a signal peptide that is not derived from the target gene (protein) itself within an organism, but other signal peptides are artificially added during gene expression. In the present disclosure, the amino acid sequence of the heterologous signal peptide comprises the amino acid sequence set forth in SEQ ID NO: 1 or SEQ ID NO: 3, or a fragment or a variant thereof.

The term "5' untranslated polynucleotide sequence" refers to the untranslated region at the 5' end of pre-mRNA or mature mRNA. For example, in mature mRNA, the 5'-UTR typically has a 7-methylguanosine cap at its 5' end and participates in multiple processes, such as splicing, polyadenylation, export of mRNA to the cytoplasm, recognition of the 5' end by the translation machinery, and protection of mRNA from degradation.

The term "3' untranslated polynucleotide sequence" refers to the untranslated region at the 3' end of pre-mRNA or mature mRNA. For example, in mature mRNA, this region contains a poly(A) tail and is known to have various functions in mRNA stability, initiation of translation, and mRNA export.

The term "promoter" refers to a genetic element that binds to proteins such as transcription factors and controls the initiation time and extent of expression of gene transcription. The CMV promoter refers to a strong promoter derived from human cytomegalovirus (CMV). In the present disclosure, the promoter includes, but is not limited to, an EF1a promoter, a CMV promoter, a CAG promoter, a PKG promoter, a CBA promoter, and a tissue-specific promoter.

The term "PolyA (pA)" refers to polyadenylic acid, which plays an important role in maintaining mRNA stability and preventing degradation thereof during extranuclear transport and translation of mRNA. Mammalian expression plasmids typically include a transcription terminator and a poly(A) sequence downstream of the target gene to promote polyadenylation and transcription termination. Poly(A) may be AATAAA. The AATAAA sequence may be replaced by other hexanucleotide sequences homologous to AATAAA and capable of signaling polyadenylation, including ATTAAA, AGTAAA, CATAAA, TATAAA, GATAAA, ACTAAA, AATATA, AAGAAA, AATAAT, AAAAAA, AATGAA, AATCAA, AACAAA, AATCAA, AATAAC, AATAGA, AATTAA, or AATAAG (see, e.g., WO 06/12414). In some embodiments, the poly(A) signal sequence may be a synthetic polyadenylation site (see, e.g., Genes Dev. 3(7): 1019-1025, 1989). Other examples of poly(A) signal sequences are known in the art.

The terms "sequence identity" and "identity" are used interchangeably and refer to the number of identical nucleotide residues or amino acid residues between two sequences when maximum correspondence is achieved over a specified comparison window by sequence alignment. The percentage of sequence identity is a value determined by comparing sequences (e.g., nucleic acid sequences or amino acid sequences) that are optimally aligned over a comparison window, wherein a portion of one sequence within the comparison window may include additions or deletions (i.e., gaps) relative to a reference sequence (which does not include additions or deletions) so as to achieve optimal alignment between the two sequences. The percentage is calculated by determining the number of positions at which identical nucleotide or amino acid residues occur between the two sequences to obtain the number of matched positions, dividing the number of matched positions by the total number of positions in the comparison window, and multiplying the result by 100 to yield the percentage sequence identity. Methods for aligning sequences for comparison are well known. Various programs and alignment algorithms are described in the following references, for example: Smith and Waterman (1981) Adv.Appl.Math.2:482; Needleman and Wunsch (1970) J.Mol.Biol.48:443; Pearson and Lipman (1988) Proc.Natl.Acad.Sci.U.S.A.85:2444; Higgins and Sharp (1988) Gene 73:237-44; Higgins and Sharp (1989) CABIOS 5:151-3; Corpetet al. (1988) Nucleic Acids Res.16:10881-90; Huang et al. (1992) Comp.Appl.Biosci.8:155-65; Pearson et al. (1994) Methods Mol.Biol.24:307-31; Tatiana et al. (1999) FEMS Microbiol.Lett.174:247-50.

The term "linked in tandem" refers to the linking of two or more polypeptides in an amino-terminus to carboxyl-terminus order. In the present disclosure, linking in tandem comprises linking the C-terminus of the first signal peptide to the N-terminus of the second signal peptide.

The term "operably linked" refers to elements or structures in a nucleic acid sequence that are linked by functionality rather than physical position. These elements or structures are capable of or characterized by performing the desired operation. Those of ordinary skill in the art recognize that elements or structures in a nucleic acid sequence are not required to be operably linked in tandem or adjacent order. "Operably linked" may include a situation in which a selected nucleotide sequence and a regulatory nucleotide sequence are covalently linked in a manner that expression of the nucleotide coding sequence is placed under the influence or control of the regulatory sequence. Therefore, if the regulatory sequence is capable of affecting transcription of part or all of the nucleotide coding sequence encoding the selected nucleotide sequence, the regulatory sequence is operably linked to the selected nucleotide sequence. Where appropriate, the resulting transcript may then be translated into a desired protein or polypeptide. In the present disclosure, the promoter, the polynucleotide sequence encoding the first signal peptide, the polynucleotide sequence encoding the second signal peptide, the sequence encoding the coagulation factor VIII, and the 3' untranslated polynucleotide sequence are operably linked.

### Example 1. F8 Gene Expression Vectors Carrying Different Signal Peptides

Three heterologous signal peptides (SPs) were selected, and each of the heterologous signal peptides was ligated to the N-terminus of a codon-optimized B-domain-deleted F8 gene (hereinafter referred to as BDDF8-CO, encoding FVIII protein) to improve expression and secretion of FVIII. Meanwhile, constructs were generated with or without the endogenous signal peptide of the F8 gene. Nucleotide sequences encoding the structures described above were respectively inserted into the multiple cloning site of a Psmart-hCkan expression vector by Sangon Biotech (Shanghai) Co., Ltd., thereby constructing: a BDDF8-CO expression plasmid comprising only the endogenous signal peptide; BDDF8-CO expression plasmids respectively guided by three different heterologous signal peptides and comprising only heterologous signal peptides (without the endogenous signal peptide); and BDDF8-CO expression plasmids respectively guided by three different heterologous signal peptides and simultaneously comprising the endogenous signal peptide (dual signal peptide).

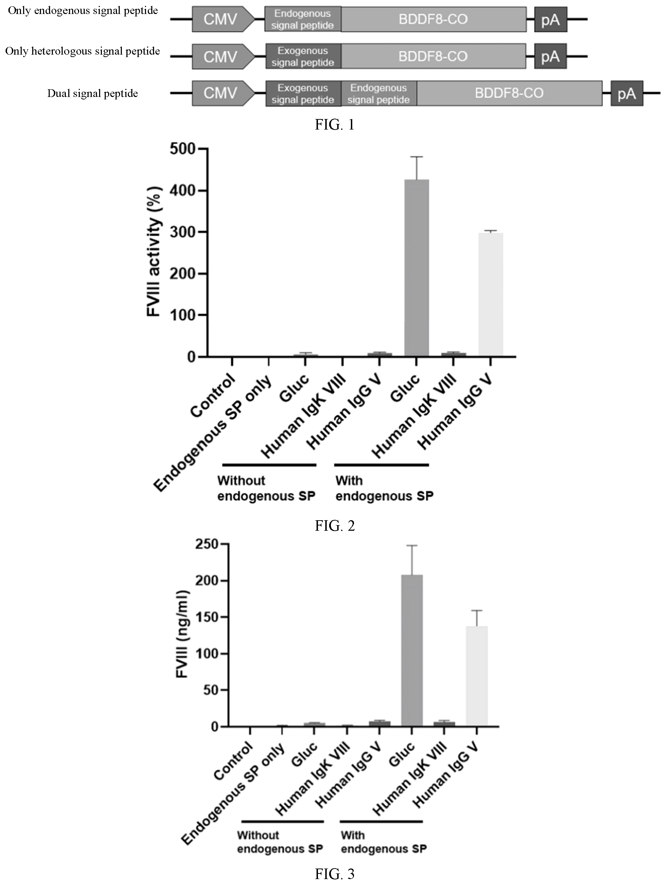

The two signal peptides were directly linked, and the signal peptide and BDDF8-CO were directly linked. The CMV promoter and the signal peptide were linked via a PacI endonuclease recognition sequence (TTAATTAA), and BDDF8-CO and polyA (pA) were linked via a NotI endonuclease recognition sequence (GCGGCCGC). Human embryonic kidney cells (HEK-293T cells) were transfected with the plasmids described above, and the expression and secretion of the target protein FVIII were detected. Schematic diagrams of expression cassettes of BDDF8-CO comprising only the endogenous signal peptide, BDDF8-CO comprising only the heterologous signal peptide, and BDDF8-CO guided by the heterologous signal peptide and simultaneously comprising the endogenous signal peptide (dual signal peptide) are shown in FIG. 1.

The nucleotide sequences of the heterologous signal peptides are as follows:
Gaussia luciferase (Gluc) (SEQ ID NO: 1):
   ATGGGCGTGAAGGTGCTGTTTGCCCTGATTTGCATCGCCGTGGCCGAGGCC;
Human IgK VIII (SEQ ID NO: 2): 
ATGGACATGAGAGTGCCCGCCCAGCTGCTGGGCCTGCTGCTGCTGTGGCTGAGGGGAG
CTAGATGT;
Human IgG V (SEQ ID NO: 3):
   ATGGACTGGACCTGGCGGTTCCTGTTTGTGGTGGCCGCCGCCACCGGCGTGCAGAGC.

The amino acid sequences of the heterologous signal peptides are as follows:
Gaussia luciferase (Gluc) (SEQ ID NO: 4): MGVKVLFALICIAVAEA;
Human IgK VIII (SEQ ID NO: 5): MDMRVPAQLLGLLLLWLRGARC;
Human IgG V (SEQ ID NO: 6): MDWTWRFLFVVAAATGVQS.

The nucleotide sequence of the endogenous signal peptide of BDDF8-CO is as follows (SEQ ID NO: 7):
ATGCAGATTGAGCTGAGCACCTGCTTCTTCCTGTGCCTGCTGAGGTTCTGCTTCTCT.

The amino acid sequence of the endogenous signal peptide of BDDF8-CO is as follows (SEQ ID NO: 8):
MQIELSTCFFLCLLRFCFS.

The nucleotide sequence of BDDF8-CO (without the endogenous signal peptide) is as follows (SEQ ID NO: 9):

GCCACCAGGAGATACTACCTGGGGGCTGTGGAGCTGAGCTGGGACTACATGCAGTCTG
ACCTGGGGGAGCTGCCTGTGGATGCCAGGTTCCCCCCCAGAGTGCCCAAGAGCTTCCC
CTTCAACACCTCTGTGGTGTACAAGAAGACCCTGTTTGTGGAGTTCACTGACCACCTGT
TCAACATTGCCAAGCCCAGGCCCCCCTGGATGGGCCTGCTGGGCCCCACCATCCAGGC
TGAGGTGTATGACACTGTGGTGATCACCCTGAAGAACATGGCCAGCCACCCTGTGAGC
CTGCATGCTGTGGGGGTGAGCTACTGGAAGGCCTCTGAGGGGGCTGAGTATGATGACC
AGACCAGCCAGAGGGAGAAGGAGGATGACAAGGTGTTCCCTGGGGGCAGCCACACCT
ATGTGTGGCAGGTGCTGAAGGAGAATGGCCCCATGGCCTCTGACCCCCTGTGCCTGAC
CTACAGCTACCTGAGCCATGTGGACCTGGTGAAGGACCTGAACTCTGGCCTGATTGGG
GCCCTGCTGGTGTGCAGGGAGGGCAGCCTGGCCAAGGAGAAGACCCAGACCCTGCAC
AAGTTCATCCTGCTGTTTGCTGTGTTTGATGAGGGCAAGAGCTGGCACTCTGAAACCAA
GAACAGCCTGATGCAGGACAGGGATGCTGCCTCTGCCAGGGCCTGGCCCAAGATGCAC
ACTGTGAATGGCTATGTGAACAGGAGCCTGCCTGGCCTGATTGGCTGCCACAGGAAGT

CTGTGTACTGGCATGTGATTGGCATGGGCACCACCCCTGAGGTGCACAGCATCTTCCTG
GAGGGCCACACCTTCCTGGTCAGGAACCACAGGCAGGCCAGCCTGGAGATCAGCCCCA
TCACCTTCCTGACTGCCCAGACCCTGCTGATGGACCTGGGCCAGTTCCTGCTGTTCTGC
CACATCAGCAGCCACCAGCATGATGGCATGGAGGCCTATGTGAAGGTGGACAGCTGCC
CTGAGGAGCCCCAGCTGAGGATGAAGAACAATGAGGAGGCTGAGGACTATGATGATG
ACCTGACTGACTCTGAGATGGATGTGGTGAGGTTTGATGATGACAACAGCCCCAGCTT
CATCCAGATCAGGTCTGTGGCCAAGAAGCACCCCAAGACCTGGGTGCACTACATTGCT
GCTGAGGAGGAGGACTGGGACTATGCCCCCCTGGTGCTGGCCCCTGATGACAGGAGCT
ACAAGAGCCAGTACCTGAACAATGGCCCCCAGAGGATTGGCAGGAAGTACAAGAAGG
TCAGGTTCATGGCCTACACTGATGAAACCTTCAAGACCAGGGAGGCCATCCAGCATGA
GTCTGGCATCCTGGGCCCCCTGCTGTATGGGGAGGTGGGGGACACCCTGCTGATCATCT
TCAAGAACCAGGCCAGCAGGCCCTACAACATCTACCCCCATGGCATCACTGATGTGAG
GCCCCTGTACAGCAGGAGGCTGCCCAAGGGGGTGAAGCACCTGAAGGACTTCCCCATC
CTGCCTGGGGAGATCTTCAAGTACAAGTGGACTGTGACTGTGGAGGATGGCCCCACCA
AGTCTGACCCCAGGTGCCTGACCAGATACTACAGCAGCTTTGTGAACATGGAGAGGGA
CCTGGCCTCTGGCCTGATTGGCCCCCTGCTGATCTGCTACAAGGAGTCTGTGGACCAGA
GGGGCAACCAGATCATGTCTGACAAGAGGAATGTGATCCTGTTCTCTGTGTTTGATGA
GAACAGGAGCTGGTACCTGACTGAGAACATCCAGAGGTTCCTGCCCAACCCTGCTGGG
GTGCAGCTGGAGGACCCTGAGTTCCAGGCCAGCAACATCATGCACAGCATCAATGGCT
ATGTGTTTGACAGCCTGCAGCTGTCTGTGTGCCTGCATGAGGTGGCCTACTGGTACATC
CTGAGCATTGGGGCCCAGACTGACTTCCTGTCTGTGTTCTTCTCTGGCTACACCTTCAA
GCACAAGATGGTGTATGAGGACACCCTGACCCTGTTCCCCTTCTCTGGGGAGACTGTGT
TCATGAGCATGGAGAACCCTGGCCTGTGGATTCTGGGCTGCCACAACTCTGACTTCAG
GAACAGGGGCATGACTGCCCTGCTGAAAGTCTCCAGCTGTGACAAGAACACTGGGGAC
TACTATGAGGACAGCTATGAGGACATCTCTGCCTACCTGCTGAGCAAGAACAATGCCA
TTGAGCCCAGGAGCTTCAGCCAGAACCCCCCAGTGCTGAAGAGGCACCAGAGGGAGA
TCACCAGGACCACCCTGCAGTCTGACCAGGAGGAGATTGACTATGATGACACCATCTC
TGTGGAGATGAAGAAGGAGGACTTTGACATCTACGACGAGGACGAGAACCAGAGCCC
CAGGAGCTTCCAGAAGAAGACCAGGCACTACTTCATTGCTGCTGTGGAGAGGCTGTGG
GACTATGGCATGAGCAGCAGCCCCCATGTGCTGAGGAACAGGGCCCAGTCTGGCTCTG
TGCCCCAGTTCAAGAAGGTGGTGTTCCAGGAGTTCACTGATGGCAGCTTCACCCAGCC
CCTGTACAGAGGGGAGCTGAATGAGCACCTGGGCCTGCTGGGCCCCTACATCAGGGCT
GAGGTGGAGGACAACATCATGGTGACCTTCAGGAACCAGGCCAGCAGGCCCTACAGCT
TCTACAGCAGCCTGATCAGCTATGAGGAGGACCAGAGGCAGGGGGCTGAGCCCAGGA

AGAACTTTGTGAAGCCCAATGAAACCAAGACCTACTTCTGGAAGGTGCAGCACCACAT
GGCCCCCACCAAGGATGAGTTTGACTGCAAGGCCTGGGCCTACTTCTCTGATGTGGAC
CTGGAGAAGGATGTGCACTCTGGCCTGATTGGCCCCCTGCTGGTGTGCCACACCAACA
CCCTGAACCCTGCCCATGGCAGGCAGGTGACTGTGCAGGAGTTTGCCCTGTTCTTCACC
ATCTTTGATGAAACCAAGAGCTGGTACTTCACTGAGAACATGGAGAGGAACTGCAGGG
CCCCCTGCAACATCCAGATGGAGGACCCCACCTTCAAGGAGAACTACAGGTTCCATGC
CATCAATGGCTACATCATGGACACCCTGCCTGGCCTGGTGATGGCCCAGGACCAGAGG
ATCAGGTGGTACCTGCTGAGCATGGGCAGCAATGAGAACATCCACAGCATCCACTTCT
CTGGCCATGTGTTCACTGTGAGGAAGAAGGAGGAGTACAAGATGGCCCTGTACAACCT
GTACCCTGGGGTGTTTGAGACTGTGGAGATGCTGCCCAGCAAGGCTGGCATCTGGAGG
GTGGAGTGCCTGATTGGGGAGCACCTGCATGCTGGCATGAGCACCCTGTTCCTGGTGT
ACAGCAACAAGTGCCAGACCCCCCTGGGCATGGCCTCTGGCCACATCAGGGACTTCCA
GATCACTGCCTCTGGCCAGTATGGCCAGTGGGCCCCCAAGCTGGCCAGGCTGCACTAC
TCTGGCAGCATCAATGCCTGGAGCACCAAGGAGCCCTTCAGCTGGATCAAGGTGGACC
TGCTGGCCCCCATGATCATCCATGGCATCAAGACCCAGGGGGCCAGGCAGAAGTTCAG
CAGCCTGTACATCAGCCAGTTCATCATCATGTACAGCCTGGATGGCAAGAAGTGGCAG
ACCTACAGGGGCAACAGCACTGGCACCCTGATGGTGTTCTTTGGCAATGTGGACAGCT
CTGGCATCAAGCACAACATCTTCAACCCCCCCATCATTGCCAGATACATCAGGCTGCAC
CCCACCCACTACAGCATCAGGAGCACCCTGAGGATGGAGCTGATGGGCTGTGACCTGA
ACAGCTGCAGCATGCCCCTGGGCATGGAGAGCAAGGCCATCTCTGATGCCCAGATCAC
TGCCAGCAGCTACTTCACCAACATGTTTGCCACCTGGAGCCCCAGCAAGGCCAGGCTG
CACCTGCAGGGCAGGAGCAATGCCTGGAGGCCCCAGGTCAACAACCCCAAGGAGTGG
CTGCAGGTGGACTTCCAGAAGACCATGAAGGTGACTGGGGTGACCACCCAGGGGGTG
AAGAGCCTGCTGACCAGCATGTATGTGAAGGAGTTCCTGATCAGCAGCAGCCAGGATG
GCCACCAGTGGACCCTGTTCTTCCAGAATGGCAAGGTGAAGGTGTTCCAGGGCAACCA
GGACAGCTTCACCCCTGTGGTGAACAGCCTGGACCCCCCCCTGCTGACCAGATACCTG
AGGATTCACCCCCAGAGCTGGGTGCACCAGATTGCCCTGAGGATGGAGGTGCTGGGCT
GTGAGGCCCAGGACCTGTACTGA.

The amino acid sequence of BDDF8-CO (without the endogenous signal peptide) is as follows (SEQ ID NO: 10):

ATRRYYLGAVELSWDYMQSDLGELPVDARFPPRVPKSFPFNTSVVYKKTLFVEFTDHLFNI
AKPRPPWMGLLGPTIQAEVYDTVVITLKNMASHPVSLHAVGVSYWKASEGAEYDDQTSQ
REKEDDKVFPGGSHTYVWQVLKENGPMASDPLCLTYSYLSHVDLVKDLNSGLIGALLVCR
EGSLAKEKTQTLHKFILLFAVFDEGKSWHSETKNSLMQDRDAASARAWPKMHTVNGYVN

RSLPGLIGCHRKSVYWHVIGMGTTPEVHSIFLEGHTFLVRNHRQASLEISPITFLTAQTLLMD
LGQFLLFCHISSHQHDGMEAYVKVDSCPEEPQLRMKNNEEAEDYDDDLTDSEMDVVRFD
DDNSPSFIQIRSVAKKHPKTWVHYIAAEEEDWDYAPLVLAPDDRSYKSQYLNNGPQRIGRK
YKKVRFMAYTDETFKTREAIQHESGILGPLLYGEVGDTLLIIFKNQASRPYNIYPHGITDVRP
LYSRRLPKGVKHLKDFPILPGEIFKYKWTVTVEDGPTKSDPRCLTRYYSSFVNMERDLASG
LIGPLLICYKESVDQRGNQIMSDKRNVILFSVFDENRSWYLTENIQRFLPNPAGVQLEDPEF
QASNIMHSINGYVFDSLQLSVCLHEVAYWYILSIGAQTDFLSVFFSGYTFKHKMVYEDTLT
LFPFSGETVFMSMENPGLWILGCHNSDFRNRGMTALLKVSSCDKNTGDYYEDSYEDISAY
LLSKNNAIEPRSFSQNPPVLKRHQREITRTTLQSDQEEIDYDDTISVEMKKEDFDIYDEDENQ
SPRSFQKKTRHYFIAAVERLWDYGMSSSPHVLRNRAQSGSVPQFKKVVFQEFTDGSFTQPL
YRGELNEHLGLLGPYIRAEVEDNIMVTFRNQASRPYSFYSSLISYEEDQRQGAEPRKNFVKP
NETKTYFWKVQHHMAPTKDEFDCKAWAYFSDVDLEKDVHSGLIGPLLVCHTNTLNPAHG
RQVTVQEFALFFTIFDETKSWYFTENMERNCRAPCNIQMEDPTFKENYRFHAINGYIMDTL
PGLVMAQDQRIRWYLLSMGSNENIHSIHFSGHVFTVRKKEEYKMALYNLYPGVFETVEML
PSKAGIWRVECLIGEHLHAGMSTLFLVYSNKCQTPLGMASGHIRDFQITASGQYGQWAPK
LARLHYSGSINAWSTKEPFSWIKVDLLAPMIIHGIKTQGARQKFSSLYISQFIIMYSLDGKKW
QTYRGNSTGTLMVFFGNVDSSGIKHNIFNPPIIARYIRLHPTHYSIRSTLRMELMGCDLNSCS
MPLGMESKAISDAQITASSYFTNMFATWSPSKARLHLQGRSNAWRPQVNNPKEWLQVDF
QKTMKVTGVTTQGVKSLLTSMYVKEFLISSSQDGHQWTLFFQNGKVKVFQGNQDSFTPV
VNSLDPPLLTRYLRIHPQSWVHQIALRMEVLGCEAQDLY.

The amino acid sequence of the Gluc dual signal peptide is as follows (SEQ ID NO: 11):
MGVKVLFALICIAVAEAMQIELSTCFFLCLLRFCFS.

The amino acid sequence of the combination of the Gluc dual signal peptide and the F8 expression cassette is as follows (SEQ ID NO: 12):

MGVKVLFALICIAVAEAMQIELSTCFFLCLLRFCFSATRRYYLGAVELSWDYMQSDLGELP
VDARFPPRVPKSFPFNTSVVYKKTLFVEFTDHLFNIAKPRPPWMGLLGPTIQAEVYDTVVIT
LKNMASHPVSLHAVGVSYWKASEGAEYDDQTSQREKEDDKVFPGGSHTYVWQVLKENG
PMASDPLCLTYSYLSHVDLVKDLNSGLIGALLVCREGSLAKEKTQTLHKFILLFAVFDEGK
SWHSETKNSLMQDRDAASARAWPKMHTVNGYVNRSLPGLIGCHRKSVYWHVIGMGTTP
EVHSIFLEGHTFLVRNHRQASLEISPITFLTAQTLLMDLGQFLLFCHISSHQHDGMEAYVKV
DSCPEEPQLRMKNNEEAEDYDDDLTDSEMDVVRFDDDNSPSFIQIRSVAKKHPKTWVHYI
AAEEEDWDYAPLVLAPDDRSYKSQYLNNGPQRIGRKYKKVRFMAYTDETFKTREAIQHES
GILGPLLYGEVGDTLLIIFKNQASRPYNIYPHGITDVRPLYSRRLPKGVKHLKDFPILPGEIFK
YKWTVTVEDGPTKSDPRCLTRYYSSFVNMERDLASGLIGPLLICYKESVDQRGNQIMSDKR

NVILFSVFDENRSWYLTENIQRFLPNPAGVQLEDPEFQASNIMHSINGYVFDSLQLSVCLHE
VAYWYILSIGAQTDFLSVFFSGYTFKHKMVYEDTLTLFPFSGETVFMSMENPGLWILGCHN
SDFRNRGMTALLKVSSCDKNTGDYYEDSYEDISAYLLSKNNAIEPRSFSQNPPVLKRHQREI
TRTTLQSDQEEIDYDDTISVEMKKEDFDIYDEDENQSPRSFQKKTRHYFIAAVERLWDYGM
SSSPHVLRNRAQSGSVPQFKKVVFQEFTDGSFTQPLYRGELNEHLGLLGPYIRAEVEDNIM
VTFRNQASRPYSFYSSLISYEEDQRQGAEPRKNFVKPNETKTYFWKVQHHMAPTKDEFDC
KAWAYFSDVDLEKDVHSGLIGPLLVCHTNTLNPAHGRQVTVQEFALFFTIFDETKSWYFTE
NMERNCRAPCNIQMEDPTFKENYRFHAINGYIMDTLPGLVMAQDQRIRWYLLSMGSNENI
HSIHFSGHVFTVRKKEEYKMALYNLYPGVFETVEMLPSKAGIWRVECLIGEHLHAGMSTLF
LVYSNKCQTPLGMASGHIRDFQITASGQYGQWAPKLARLHYSGSINAWSTKEPFSWIKVD
LLAPMIIHGIKTQGARQKFSSLYISQFIIMYSLDGKKWQTYRGNSTGTLMVFFGNVDSSGIK
HNIFNPPIIARYIRLHPTHYSIRSTLRMELMGCDLNSCSMPLGMESKAISDAQITASSYFTNM
FATWSPSKARLHLQGRSNAWRPQVNNPKEWLQVDFQKTMKVTGVTTQGVKSLLTSMYV
KEFLISSSQDGHQWTLFFQNGKVKVFQGNQDSFTPVVNSLDPPLLTRYLRIHPQSWVHQIA
LRMEVLGCEAQDLY.

The nucleotide sequence of the combination of the Gluc dual signal peptide and the F8 expression cassette is as follows (SEQ ID NO: 13):

ATGGGCGTGAAGGTGCTGTTTGCCCTGATTTGCATCGCCGTGGCCGAGGCCATGCAGA
TTGAGCTGAGCACCTGCTTCTTCCTGTGCCTGCTGAGGTTCTGCTTCTCTGCCACCAGG
AGATACTACCTGGGGGCTGTGGAGCTGAGCTGGGACTACATGCAGTCTGACCTGGGGG
AGCTGCCTGTGGATGCCAGGTTCCCCCCCAGAGTGCCCAAGAGCTTCCCCTTCAACACC
TCTGTGGTGTACAAGAAGACCCTGTTTGTGGAGTTCACTGACCACCTGTTCAACATTGC
CAAGCCCAGGCCCCCCTGGATGGGCCTGCTGGGCCCCACCATCCAGGCTGAGGTGTAT
GACACTGTGGTGATCACCCTGAAGAACATGGCCAGCCACCCTGTGAGCCTGCATGCTG
TGGGGGTGAGCTACTGGAAGGCCTCTGAGGGGGCTGAGTATGATGACCAGACCAGCCA
GAGGGAGAAGGAGGATGACAAGGTGTTCCCTGGGGGCAGCCACACCTATGTGTGGCA
GGTGCTGAAGGAGAATGGCCCCATGGCCTCTGACCCCCTGTGCCTGACCTACAGCTAC
CTGAGCCATGTGGACCTGGTGAAGGACCTGAACTCTGGCCTGATTGGGGCCCTGCTGG
TGTGCAGGGAGGGCAGCCTGGCCAAGGAGAAGACCCAGACCCTGCACAAGTTCATCCT
GCTGTTTGCTGTGTTTGATGAGGGCAAGAGCTGGCACTCTGAAACCAAGAACAGCCTG
ATGCAGGACAGGGATGCTGCCTCTGCCAGGGCCTGGCCCAAGATGCACACTGTGAATG
GCTATGTGAACAGGAGCCTGCCTGGCCTGATTGGCTGCCACAGGAAGTCTGTGTACTG
GCATGTGATTGGCATGGGCACCACCCCTGAGGTGCACAGCATCTTCCTGGAGGGCCAC
ACCTTCCTGGTCAGGAACCACAGGCAGGCCAGCCTGGAGATCAGCCCCATCACCTTCC

TGACTGCCCAGACCCTGCTGATGGACCTGGGCCAGTTCCTGCTGTTCTGCCACATCAGC
AGCCACCAGCATGATGGCATGGAGGCCTATGTGAAGGTGGACAGCTGCCCTGAGGAGC
CCCAGCTGAGGATGAAGAACAATGAGGAGGCTGAGGACTATGATGATGACCTGACTG
ACTCTGAGATGGATGTGGTGAGGTTTGATGATGACAACAGCCCCAGCTTCATCCAGAT
CAGGTCTGTGGCCAAGAAGCACCCCAAGACCTGGGTGCACTACATTGCTGCTGAGGAG
GAGGACTGGGACTATGCCCCCCTGGTGCTGGCCCCTGATGACAGGAGCTACAAGAGCC
AGTACCTGAACAATGGCCCCCAGAGGATTGGCAGGAAGTACAAGAAGGTCAGGTTCAT
GGCCTACACTGATGAAACCTTCAAGACCAGGGAGGCCATCCAGCATGAGTCTGGCATC
CTGGGCCCCCTGCTGTATGGGGAGGTGGGGGACACCCTGCTGATCATCTTCAAGAACC
AGGCCAGCAGGCCCTACAACATCTACCCCCATGGCATCACTGATGTGAGGCCCCTGTA
CAGCAGGAGGCTGCCCAAGGGGGTGAAGCACCTGAAGGACTTCCCCATCCTGCCTGGG
GAGATCTTCAAGTACAAGTGGACTGTGACTGTGGAGGATGGCCCCACCAAGTCTGACC
CCAGGTGCCTGACCAGATACTACAGCAGCTTTGTGAACATGGAGAGGGACCTGGCCTC
TGGCCTGATTGGCCCCCTGCTGATCTGCTACAAGGAGTCTGTGGACCAGAGGGGCAAC
CAGATCATGTCTGACAAGAGGAATGTGATCCTGTTCTCTGTGTTTGATGAGAACAGGA
GCTGGTACCTGACTGAGAACATCCAGAGGTTCCTGCCCAACCCTGCTGGGGTGCAGCT
GGAGGACCCTGAGTTCCAGGCCAGCAACATCATGCACAGCATCAATGGCTATGTGTTT
GACAGCCTGCAGCTGTCTGTGTGCCTGCATGAGGTGGCCTACTGGTACATCCTGAGCAT
TGGGGCCCAGACTGACTTCCTGTCTGTGTTCTTCTCTGGCTACACCTTCAAGCACAAGA
TGGTGTATGAGGACACCCTGACCCTGTTCCCCTTCTCTGGGGAGACTGTGTTCATGAGC
ATGGAGAACCCTGGCCTGTGGATTCTGGGCTGCCACAACTCTGACTTCAGGAACAGGG
GCATGACTGCCCTGCTGAAAGTCTCCAGCTGTGACAAGAACACTGGGGACTACTATGA
GGACAGCTATGAGGACATCTCTGCCTACCTGCTGAGCAAGAACAATGCCATTGAGCCC
AGGAGCTTCAGCCAGAACCCCCCAGTGCTGAAGAGGCACCAGAGGGAGATCACCAGG
ACCACCCTGCAGTCTGACCAGGAGGAGATTGACTATGATGACACCATCTCTGTGGAGA
TGAAGAAGGAGGACTTTGACATCTACGACGAGGACGAGAACCAGAGCCCCAGGAGCT
TCCAGAAGAAGACCAGGCACTACTTCATTGCTGCTGTGGAGAGGCTGTGGGACTATGG
CATGAGCAGCAGCCCCCATGTGCTGAGGAACAGGGCCCAGTCTGGCTCTGTGCCCCAG
TTCAAGAAGGTGGTGTTCCAGGAGTTCACTGATGGCAGCTTCACCCAGCCCCTGTACA
GAGGGGAGCTGAATGAGCACCTGGGCCTGCTGGGCCCCTACATCAGGGCTGAGGTGGA
GGACAACATCATGGTGACCTTCAGGAACCAGGCCAGCAGGCCCTACAGCTTCTACAGC
AGCCTGATCAGCTATGAGGAGGACCAGAGGCAGGGGGCTGAGCCCAGGAAGAACTTT
GTGAAGCCCAATGAAACCAAGACCTACTTCTGGAAGGTGCAGCACCACATGGCCCCCA
CCAAGGATGAGTTTGACTGCAAGGCCTGGGCCTACTTCTCTGATGTGGACCTGGAGAA

GGATGTGCACTCTGGCCTGATTGGCCCCCTGCTGGTGTGCCACACCAACACCCTGAACC
CTGCCCATGGCAGGCAGGTGACTGTGCAGGAGTTTGCCCTGTTCTTCACCATCTTTGAT
GAAACCAAGAGCTGGTACTTCACTGAGAACATGGAGAGGAACTGCAGGGCCCCCTGC
AACATCCAGATGGAGGACCCCACCTTCAAGGAGAACTACAGGTTCCATGCCATCAATG
GCTACATCATGGACACCCTGCCTGGCCTGGTGATGGCCCAGGACCAGAGGATCAGGTG
GTACCTGCTGAGCATGGGCAGCAATGAGAACATCCACAGCATCCACTTCTCTGGCCAT
GTGTTCACTGTGAGGAAGAAGGAGGAGTACAAGATGGCCCTGTACAACCTGTACCCTG
GGGTGTTTGAGACTGTGGAGATGCTGCCCAGCAAGGCTGGCATCTGGAGGGTGGAGTG
CCTGATTGGGGAGCACCTGCATGCTGGCATGAGCACCCTGTTCCTGGTGTACAGCAAC
AAGTGCCAGACCCCCCTGGGCATGGCCTCTGGCCACATCAGGGACTTCCAGATCACTG
CCTCTGGCCAGTATGGCCAGTGGGCCCCCAAGCTGGCCAGGCTGCACTACTCTGGCAG
CATCAATGCCTGGAGCACCAAGGAGCCCTTCAGCTGGATCAAGGTGGACCTGCTGGCC
CCCATGATCATCCATGGCATCAAGACCCAGGGGGCCAGGCAGAAGTTCAGCAGCCTGT
ACATCAGCCAGTTCATCATCATGTACAGCCTGGATGGCAAGAAGTGGCAGACCTACAG
GGGCAACAGCACTGGCACCCTGATGGTGTTCTTTGGCAATGTGGACAGCTCTGGCATC
AAGCACAACATCTTCAACCCCCCCATCATTGCCAGATACATCAGGCTGCACCCCACCC
ACTACAGCATCAGGAGCACCCTGAGGATGGAGCTGATGGGCTGTGACCTGAACAGCTG
CAGCATGCCCCTGGGCATGGAGAGCAAGGCCATCTCTGATGCCCAGATCACTGCCAGC
AGCTACTTCACCAACATGTTTGCCACCTGGAGCCCCAGCAAGGCCAGGCTGCACCTGC
AGGGCAGGAGCAATGCCTGGAGGCCCCAGGTCAACAACCCCAAGGAGTGGCTGCAGG
TGGACTTCCAGAAGACCATGAAGGTGACTGGGGTGACCACCCAGGGGGTGAAGAGCC
TGCTGACCAGCATGTATGTGAAGGAGTTCCTGATCAGCAGCAGCCAGGATGGCCACCA
GTGGACCCTGTTCTTCCAGAATGGCAAGGTGAAGGTGTTCCAGGGCAACCAGGACAGC
TTCACCCCTGTGGTGAACAGCCTGGACCCCCCCCTGCTGACCAGATACCTGAGGATTCA
CCCCCAGAGCTGGGTGCACCAGATTGCCCTGAGGATGGAGGTGCTGGGCTGTGAGGCC
CAGGACCTGTACTGA.

### Example 2. Transfection of HEK-293T Cells with Signal Peptide Efficiency Verification Plasmids and Detection of FVIII Expression and Secretion Levels

### 1. Transfection of HEK-293T cells with signal peptide efficiency verification plasmids

Human embryonic kidney cell line HEK-293T (Wuhan Procell Life Science & Technology Co., Ltd., #CL-0005, hereinafter referred to as 293T cells) was cultured. When the cells reached about 80% confluence, the complete culture medium (high-glucose DMEM medium (Gibco, #C11995500BT) containing 10% FBS (Gibco, #12664025)) was discarded. The cells were gently washed twice with DPBS, and then digested with 0.05% trypsin (Gibco, #15050065) for 2 min. The digestion solution was discarded, and the cells were resuspended in a complete culture medium. A total of 4 × 10⁵ cells were seeded per well in a 12-well plate. When the confluence of 293T cells reached about 40%, 1 µg of the efficiency verification plasmid (the plasmids obtained in Example 1) was transfected into each well using lipo2000 transfection reagent (Thermo, #11668019) (the control group was a negative control), and the transfection procedure was performed in accordance with the manufacturer's instructions. After 12 h, the culture medium was replaced with 1 mL of fresh complete culture medium for continued culture. The transfection experiment was repeated 3 times for each group independently.

### 2. Detection of coagulation activity of FVIII in cell supernatant using an automated coagulation analyzer

To evaluate the effects of different signal peptides or combinations thereof on the secretion efficiency and corresponding coagulation activity of FVIII, cell culture supernatants were collected at 72 h after transfection of the efficiency verification plasmids. An automated coagulation analyzer (Sysmex, #CS-2400) was used to determine the FVIII activity percentage in the culture supernatants of each group by an intrinsic coagulation factor activity assay, in accordance with the instrument instructions and using the corresponding reagents of the instrument. The FVIII activity assay results are shown in FIG. 2.

As can be seen from FIG. 2, in the group comprising only the endogenous signal peptide (endogenous SP only), FVIII activity was almost undetectable. In the three groups comprising only heterologous signal peptides (without endogenous SP), FVIII activity was also relatively low, with all detected values not exceeding 10%. In contrast, in the group comprising both the heterologous Gluc signal peptide and the endogenous signal peptide, FVIII activity exceeded 400%, about 4 IU/mL. In the group comprising both the heterologous Human IgG V signal peptide and the endogenous signal peptide, FVIII activity was also relatively high, about 300%. Therefore, compared with a single signal peptide or other types of dual signal peptide combinations, the dual signal peptide combination composed of the heterologous Gluc signal peptide and the endogenous signal peptide achieves the optimal coagulation activity, far exceeding the coagulation effect achievable by other single signal peptides.

### 3. Detection of F8 content in cell supernatant by ELISA

To further verify the secretion levels of coagulation factor achievable by different signal peptides, cell culture supernatants were collected at 72 h after transfection. The content of factor VIII in the culture supernatants of each group was detected using a human FVIII ELISA kit (CEDARLANE, #CL20035K), in accordance with the instructions of the kit. The results of FVIII secretion levels detected by ELISA are shown in FIG. 3.

As shown in FIG. 3, in the group comprising only the endogenous signal peptide (endogenous SP only), the FVIII content was almost undetectable. In the three groups comprising only heterologous signal peptides (without endogenous SP), the FVIII content was slightly increased but remained relatively low, with all detected values not exceeding 10 ng/mL. When the heterologous signal peptide and the endogenous signal peptide were combined to guide FVIII expression, the effect was most significant. In particular, in the dual signal peptide combination group comprising both the heterologous Gluc signal peptide and the endogenous signal peptide, the FVIII expression level was significantly increased compared with that of the single signal peptide groups (comprising only the endogenous signal peptide or only the heterologous signal peptide), exceeding 200 ng/mL.

As can be seen from the above experimental results, for FVIII, due to its large protein size and the large number of amino acids constituting the protein, a single signal peptide generally fails to achieve good secretion efficiency in cells, resulting in extremely low secretion levels and coagulation activity. In the specific embodiments of the present disclosure, a dual signal peptide strategy is particularly employed to improve both secretion levels and coagulation activity. Preferably, the heterologous signal peptide is combined with the endogenous signal peptide of the protein itself to form a dual signal peptide structure, thereby improving the expression and secretion of the target protein. The specific examples of the present disclosure demonstrate, from both coagulation activity and expression level perspectives, that the dual signal peptide combination can significantly improve the expression and secretion of the target protein compared with a single heterologous signal peptide or a single endogenous signal peptide. Among them, the dual signal peptide combination comprising the heterologous Gluc signal peptide and the endogenous signal peptide exhibits the optimal effect. Finally, it should be noted that the above examples are merely intended for illustrating the technical solutions of the present disclosure rather than limiting the present disclosure. Although the present disclosure is described in detail with reference to preferred examples, it should be understood by those of ordinary skill in the art that modifications or equivalent substitutions can be made to the technical solutions of the present disclosure without departing from the spirit and scope of the technical solutions of the present disclosure, and such modifications and equivalent substitutions should fall within the scope of the claims of the present disclosure.

## Claims

1. A dual signal peptide, wherein the dual signal peptide comprise a first signal peptide and a second signal peptide, the first signal peptide comprises a heterologous signal peptide, the second signal peptide comprises an endogenous signal peptide of coagulation factor VIII, and the first signal peptide and the second signal peptide are linked in tandem.

2. The dual signal peptide according to claim 1, wherein the heterologous signal peptide comprises any available signal peptide capable of promoting the secretion of coagulation factor VIII from a cell; preferably, the heterologous signal peptide is one or both of Gaussia luciferase and IgG V.

3. The dual signal peptide according to claim 2, wherein the heterologous signal peptide comprises an amino acid sequence as follows: the amino acid sequence of the heterologous signal peptide is set forth in any one of SEQ ID NO: 4 and SEQ ID NO: 6, or an amino acid sequence having at least 95%, at least 96%, at least 97%, at least 98%, or at least 99% sequence identity to the sequence set forth in any one of SEQ ID NO: 4 and SEQ ID NO: 6 and having substantially the same activity.

4. The dual signal peptide according to claim 1, wherein the endogenous signal peptide has an amino acid sequence set forth in SEQ ID NO: 8, or an amino acid sequence having at least 95%, at least 96%, at least 97%, at least 98%, or at least 99% sequence identity to SEQ ID NO: 8 and having substantially the same activity.

5. The dual signal peptide according to claim 1, wherein the first signal peptide and the second signal peptide being linked in tandem comprises a C-terminus of the first signal peptide being linked to an N-terminus of the second signal peptide;
preferably, a spacer sequence is present or absent between the first signal peptide and the second signal peptide;
preferably, the dual signal peptide has an amino acid sequence set forth in SEQ ID NO: 11, or an amino acid sequence having at least 95%, at least 96%, at least 97%, at least 98%, or at least 99% sequence identity to SEQ ID NO: 11.

6. A polypeptide, wherein the polypeptide comprises the dual signal peptide according to any one of claims 1-5 and coagulation factor VIII,
preferably, the coagulation factor VIII comprises modified or unmodified coagulation factor VIII;
preferably, the coagulation factor VIII is selected from full-length coagulation factor VIII or a variant thereof, or B-domain-deleted coagulation factor VIII or a variant thereof; more preferably, the coagulation factor VIII comprises a BDDF8-CO variant, a BDDF8-SQ variant, a BDDF8-N222 variant, or a BDDF8-N6 variant;
preferably, the coagulation factor VIII has an amino acid sequence set forth in SEQ ID NO: 10, or an amino acid sequence having at least 95%, at least 96%, at least 97%, at least 98%, or at least 99% sequence identity to SEQ ID NO: 10 and having substantially the same activity;
preferably, the C-terminus of the dual signal peptide is linked to an N-terminus of the coagulation factor VIII;
preferably, a spacer sequence is present or absent between the dual signal peptide and the coagulation factor VIII;
preferably, the polypeptide has an amino acid sequence set forth in SEQ ID NO: 12, or an amino acid sequence having at least 95%, at least 96%, at least 97%, at least 98%, or at least 99% sequence identity to SEQ ID NO: 12.

7. A polynucleotide, wherein the polynucleotide encodes the dual signal peptide according to any one of claims 1-5 or the polypeptide according to claim 6,
preferably, the polynucleotide sequence encoding the first signal peptide is set forth in any one of SEQ ID NO: 1 and SEQ ID NO: 3, or a nucleotide sequence having at least 95%, at least 96%, at least 97%, at least 98%, or at least 99% sequence identity to the polynucleotide sequence encoding the first signal peptide set forth in any one of SEQ ID NO: 1 and SEQ ID NO: 3;
preferably, the polynucleotide sequence encoding the second signal peptide is set forth in SEQ ID NO: 7, or a nucleotide sequence having at least 95%, at least 96%, at least 97%, at least 98%, or at least 99% sequence identity to SEQ ID NO: 7;
preferably, the sequence encoding the coagulation factor VIII is set forth in SEQ ID NO: 9, or a nucleotide sequence having at least 95%, at least 96%, at least 97%, at least 98%, or at least 99% sequence identity to SEQ ID NO: 9;
preferably, the polynucleotide sequence encoding the first signal peptide, the polynucleotide sequence encoding the second signal peptide, and the sequence encoding the coagulation factor VIII are operably linked;
preferably, the polynucleotide comprises, from the 5' end to the 3' end, the polynucleotide sequence encoding the first signal peptide-the polynucleotide sequence encoding the second signal peptide-the sequence encoding the coagulation factor VIII;
preferably, the polynucleotide has a sequence set forth in SEQ ID NO: 13, or a nucleotide sequence having at least 95%, at least 96%, at least 97%, at least 98%, or at least 99% sequence identity to SEQ ID NO: 13;
preferably, the polynucleotide further comprises a promoter and a 3' untranslated polynucleotide sequence; more preferably, the 3' untranslated polynucleotide sequence comprises a PolyA tail;
preferably, the promoter, the polynucleotide, and the 3' untranslated polynucleotide sequence are operably linked.

8. An expression vector, comprising the polynucleotide according to claim 7.

9. A host cell, comprising one or more of the dual signal peptide according to any one of claims 1-5, the polypeptide according to claim 6, the polynucleotide according to claim 7, or the expression vector according to claim 8.

10. A pharmaceutical composition, comprising one or more of the dual signal peptide according to any one of claims 1-5, the polypeptide according to claim 6, the polynucleotide according to claim 7, the expression vector according to claim 8, and the host cell according to claim 9.

11. Use of the dual signal peptide according to any one of claims 1-5, the polypeptide according to claim 6, the polynucleotide according to claim 7 , the expression vector according to claim 8, the host cell according to claim 9, or the pharmaceutical composition according to claim 10 for manufacturing coagulation factor VIII or manufacturing a medicament for treating hemophilia.

12. A method for secreting coagulation factor VIII, comprising secreting coagulation factor VIII using the dual signal peptide according to any one of claims 1-5, the polypeptide according to claim 6, the polynucleotide according to claim 7 ,the expression vector according to claim 8, the host cell according to claim 9, or the pharmaceutical composition according to claim 10,
preferably, the method comprises the following steps: constructing an expression vector containing the polynucleotide encoding the dual signal peptide and the sequence encoding coagulation factor VIII, introducing the expression vector into a host cell, and culturing the host cell under conditions allowing expression and secretion of the coagulation factor VIII to obtain the coagulation factor VIII; preferably, the method comprises culturing the host cell under conditions allowing expression and secretion of the coagulation factor VIII, and isolating the coagulation factor VIII from a cell culture.
